# EUROPEAN PATENT SPECIFICATION

(11) **EP 3 228 638 B1**
(45) Date of publication and mention of the grant of the patent: **10.04.2019**
(21) Application number: 17154198.0
(22) Date of filing: 01.02.2017
(51) Int. Cl.: C08F 2/48, A61L 29/04, A61M 25/00, C08C 19/28, C08J 7/00, C08F 291/00

(54) **SURFACE MODIFICATION METHOD**
OBERFLÄCHENMODIFIKATIONSVERFAHREN
PROCÉDÉ DE MODIFICATION DE SURFACE

(30) Priority: 04.04.2016 JP 2016075395
(43) Date of publication of application: 11.10.2017
(73) Proprietor: Sumitomo Rubber Industries, Ltd., Kobe-shi, Hyogo-ken 651-0072 (JP)
(72) Inventor: KOSAKA, Shunsuke, Kobe-shi, Hyogo 651-0072 (JP); MINAGAWA, Yasuhisa, Kobe-shi, Hyogo 651-0072 (JP)
(74) Representative: Manitz Finsterwald Patent- und Rechtsanwaltspartnerschaft mbB

(56) References cited:
- EP-A1- 2 664 627
- EP-A1- 2 692 785
- EP-A1- 2 743 294
- EP-A1- 2 796 155
- EP-A1- 3 000 842
- EP-A2- 2 623 335
- WO-A1-2015/087656
- JP-A- 2014 031 428

## Description

### TECHNICAL FIELD

The present invention relates to surface modification methods capable of providing surfaces which exhibit lubricity when wetted. The present invention also relates to surface-modified elastic bodies, e.g. surface-modified medical devices or catheters, at least a part of whose surface has been modified by the modification methods.

### BACKGROUND ART

Catheters used in medical and other fields, such as vascular catheters or urethral catheters for urethral catheterization, are inserted into blood vessels, digestive tracts, tracheae, bile ducts, or ureters and used in aqueous solutions like blood or body fluids. Therefore, they need to be able to be smoothly inserted without damaging tissues. Additionally, since catheters are inserted over guidewires, they are also required to have inner surfaces with low friction.

For this reason, low friction lubricants or lubricating layers are applied to or coated on not only the outer surfaces but also the inner surfaces of catheters before use (see Patent Literatures 1 to 3), but their lubricity is insufficient. Another problem is that their lubricity is reduced due to e.g. their separation or peeling during movement within a vessel or tract because these layers are not chemically fixed to the catheter surfaces.
WO 2015/087656 A1 discloses that a catheter may be modified using photopolymerization. EP 3 000 842 A1 and EP 2 796 155 A1 disclose that photopolymerization can be used for modifying a surface of a tube.

### CITATION LIST

### PATENT LITERATURE

Patent Literature 1: JP 2011-188908 A
Patent Literature 2: JP 2009-518479 T
Patent Literature 3: JP H07-100744 B

### SUMMARY OF INVENTION

### TECHNICAL PROBLEM

The present invention aims to solve the above problems and provide methods for surface-modifying a tubular object of a rubber vulcanizate or a thermoplastic elastomer. The methods allow these objects to have at least a lubricating inner surface layer chemically fixed thereon, instead of having a resin coating which has drawbacks such as a reduction in lubricity due to e.g. separation or peeling of the coating during movement within a vessel or tract.

### SOLUTION TO PROBLEM

A first aspect of the present invention relates to a method of surface-modifying a tubular object made of a rubber vulcanizate or a thermoplastic elastomer whose side wall may have an opening, the method including: step 1 of forming polymerization initiation points on at least an outer surface of the object; and step 2 of irradiating the outer surface of the object with ultraviolet light having a wavelength of 300 to 400 nm to radically polymerize a monomer using radicals generated from the polymerization initiation points to grow polymer chains on at least an inner surface of the object, wherein the object is an opaque tubular object made of a rubber vulcanizate or a thermoplastic elastomer, and in step 1, polymerization initiation points are formed on the inner surface as well as the outer surface of the object.

A second aspect of the present invention relates to a method of surface-modifying a tubular object made of a rubber vulcanizate or a thermoplastic elastomer, the method including step I of irradiating an outer surface of the object with ultraviolet light having a wavelength of 300 to 400 nm in the presence of a photopolymerization initiator to radically polymerize a monomer to grow polymer chains on at least an inner surface of the object, wherein the object is an opaque tubular object made of a rubber vulcanizate or a thermoplastic elastomer, and in step I, polymerization initiation points are formed on the inner surface as well as the outer surface of the object.

In the first aspect of the present invention, the object is an opaque tubular object made of a rubber vulcanizate or a thermoplastic elastomer, and in step 1, polymerization initiation points are formed on the inner surface as well as the outer surface of the object.

In the first and second aspects of the present invention, preferably, the object is an opaque tubular object made of a rubber vulcanizate or a thermoplastic elastomer, and in step 2 or step I, polymer chains are grown on the outer surface as well as the inner surface of the object.

The photopolymerization initiator is preferably at least one of a benzophenone compound or a thioxanthone compound.

The monomer is preferably at least one selected from the group consisting of alkali metal-containing monomers, halogen-containing monomers, and zwitterionic monomers.

At least a part of the inner surface of the object is preferably modified to impart lubricity in the presence of water.

The object is preferably a catheter.

### ADVANTAGEOUS EFFECTS OF INVENTION

The present invention provides surface modification methods such as a method for surface-modifying a tubular object made of a rubber vulcanizate or a thermoplastic elastomer whose side wall may have an opening, the method including: step 1 of forming polymerization initiation points on at least the outer surface of the object; and step 2 of irradiating the outer surface of the object with ultraviolet light having a wavelength of 300 to 400 nm to radically polymerize a monomer using radicals generated from the polymerization initiation points to grow polymer chains on at least the inner surface of the object. The tubular objects surface-modified by the methods have a polymer having lubricity fixed to the inner surface, and thus the inner surface is provided with excellent lubricity and excellent lubricant durability to repeated movements, i.e. a durability such that there will be little reduction in lubricity. Accordingly, by forming polymer chains on the inner surface of the objects to be modified according to the methods of the present invention, it is possible to produce surface-modified elastic bodies, e.g. surface-modified catheters, which are excellent in these properties.

### BRIEF DESCRIPTION OF DRAWINGS

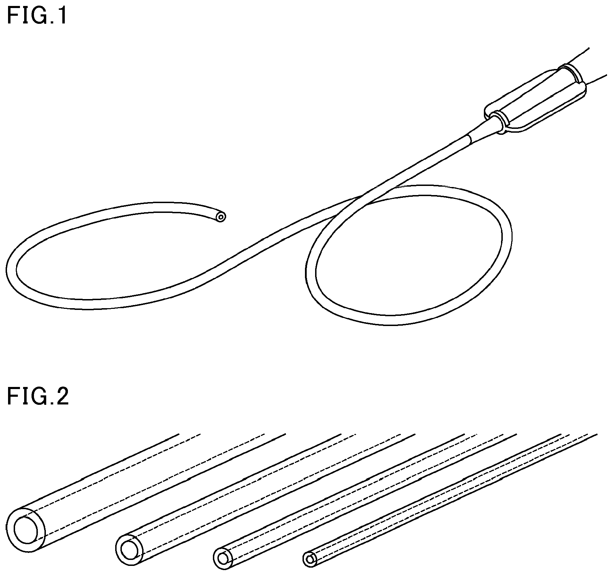

Fig. 1 is an exemplary schematic view of a vascular catheter.
Fig. 2 shows exemplary schematic views of catheters having different diameters.
Fig. 3 shows exemplary schematic views of tubular objects to be modified whose side wall has or does not have an opening.
Fig. 4 shows exemplary cross-sectional views taken in the tube radial direction of tubular objects to be modified whose side wall has or does not have an opening.

### DESCRIPTION OF EMBODIMENTS

The present invention provides a method for surface-modifying a tubular object made of a rubber vulcanizate or a thermoplastic elastomer whose side wall may have an opening, the method including: step 1 of forming polymerization initiation points on at least the outer surface of the object; and step 2 of irradiating the outer surface of the object with ultraviolet light having a wavelength of 300 to 400 nm to radically polymerize a monomer using radicals generated from the polymerization initiation points to grow polymer chains on at least the inner surface of the object.

In step 1, polymerization initiation points are formed on the outer surface and optionally the inner surface of a vulcanized rubber tube whose side wall may have an opening or a formed thermoplastic elastomer tube whose side wall may have an opening, each of which is an object to be modified. For example, the step 1 may be carried out by adsorbing a photopolymerization initiator onto the outer surface and optionally the inner surface of the object to form polymerization initiation points, or by adsorbing a photopolymerization initiator onto the outer surface and optionally the inner surface of the object, followed by irradiation with ultraviolet light having a wavelength of 300 to 400 nm to form polymerization initiation points from the photopolymerization initiator on the outer surface and optionally the inner surface.

Examples of the thermoplastic elastomer include nylon, polyester, polyurethane, polypropylene, acrylonitrile-butadiene-styrene copolymer resin (ABS), fluororesins such as polytetrafluoroethylene, and dynamically crosslinked thermoplastic elastomers prepared from these elastomers. Examples of nylon include nylon 6, nylon 66, nylon 11, and nylon 12. Preferred dynamically crosslinked thermoplastic elastomers are those obtained by dynamically crosslinking halogenated butyl rubbers in thermoplastic elastomers. Preferred examples of the thermoplastic elastomers include nylon, polyurethane, polypropylene, and styrene-isobutylene-styrene block copolymer (SIBS).

Examples of the rubber vulcanizate include natural rubber, deproteinized natural rubber, styrene-butadiene rubber, polybutadiene rubber, polyisoprene rubber, silicone rubber, and butyl rubber and halogenated butyl rubbers which have a degree of unsaturation of a few percent of isoprene units.

The vulcanization conditions of the rubber may be selected appropriately. The vulcanization temperature of the rubber is preferably 140°C or higher, more preferably 170°C or higher, still more preferably 175°C or higher.

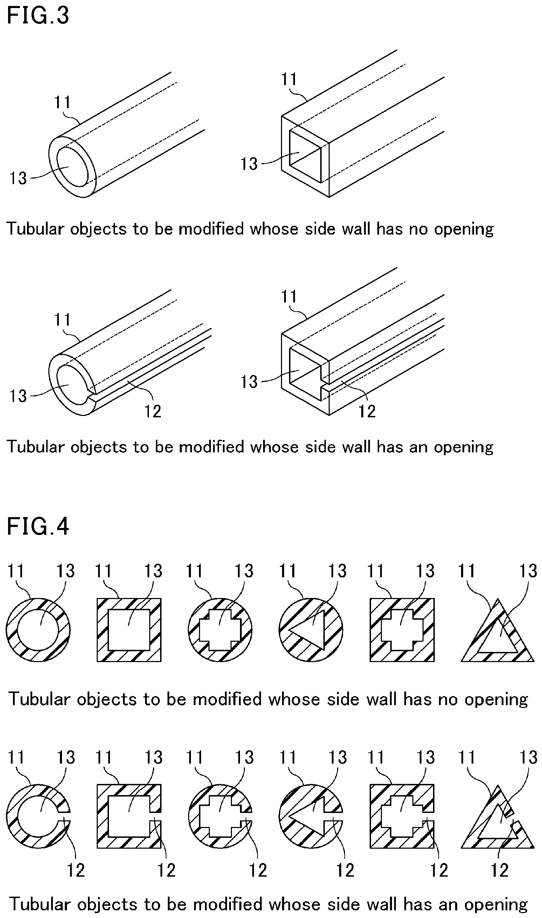

The tubular object made of a rubber vulcanizate or a thermoplastic elastomer may have either a tubular shape whose side wall has no opening (closed tubular shape) or a tubular shape whose side wall has an opening(s) (open tubular shape) . Fig. 3 shows examples of tubular objects to be modified, including tubular objects whose side wall 11 has no opening and tubular objects whose side wall 11 has an opening 12 (objects with the opening 12 extending in the tube longitudinal direction). The shape of the opening 12 is not particularly limited and examples include embodiments in which an opening is formed extending throughout the length of a tube as shown in Fig. 3, and embodiments in which one or more openings having a substantially circular or rectangular shape or other shapes is/are formed on the side wall of a tubular object.

Fig. 4 shows cross-sectional views taken in the tube radial direction of tubular objects to be modified whose side wall has or does not have an opening. As shown in Fig. 4, the interior shape of the tubular object (the shape of the interior 13) is not particularly limited and may be any cavity shape that allows for flowing (penetration) in the tube longitudinal direction.

The object to be modified is opaque. In particular, in the case where the outer surface of an opaque tubular object is irradiated with light from outside, radicals are generated from the photopolymerization initiator on the outer surface, while no light reaches the inner surface. Thus, even when a photopolymerization initiator is present on the inner surface, no radicals are generated from the initiator. In the present invention, however, as described later, when the outer surface of an opaque tubular object is irradiated with light in step 2, radicals generated on the outer surface can be transferred to the inner surface, whereby polymer chains can be formed also on the inner surface. Thus, the method of the present invention can be suitably applied particularly to opaque (e.g. black) objects to be modified.

Examples of the photopolymerization initiator include carbonyl compounds, organic sulfur compounds such as tetraethylthiuram disulfide, persulfides, redox compounds, azo compounds, diazo compounds, halogen compounds, and photoreducing dyes. Preferred among these are carbonyl compounds.

Preferred among carbonyl compounds serving as photopolymerization initiators are benzophenone and derivatives thereof (benzophenone compounds). For example, suitable are benzophenone compounds represented by the following formula: 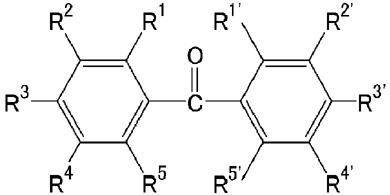 wherein R¹ to R⁵ and R^{1'} to R^{5'} are the same as or different from one another and each represent a hydrogen atom, an alkyl group, a halogen (fluorine, chlorine, bromine, or iodine), a hydroxy group, a primary to tertiary amino group, a mercapto group, or a hydrocarbon group that may contain an oxygen atom, a nitrogen atom, or a sulfur atom, and any two adjacent groups of R¹ to R⁵ and R^{1'} to R^{5'} may be joined to each other to form a ring together with the carbon atoms to which they are attached.

Specific examples of the benzophenone compounds include benzophenone, xanthone, 9-fluorenone, 2,4-dichlorobenzophenone, methyl o-benzoylbenzoate, 4,4'-bis(dimethylamino)benzophenone, and 4,4'-bis(diethylamino)benzophenone. Particularly preferred among these are benzophenone, xanthone, and 9-fluorenone as these compounds contribute to forming polymer brushes well.

The photopolymerization initiator may also suitably be a thioxanthone compound because it provides a high polymerization rate and can easily be adsorbed onto and/or reacted with rubber or the like. Suitable examples include compounds represented by the following formula: 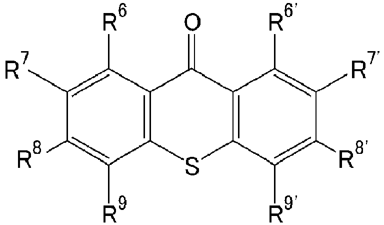 wherein R⁶ to R⁹ and R^{6'} to R^{9'} are the same as or different from one another and each represent a hydrogen atom, a halogen atom, an alkyl group, a cyclic alkyl group, an aryl group, an alkenyl group, an alkoxy group, or an aryloxy group.

Examples of thioxanthone compounds represented by the above formula include thioxanthone, 2-isopropylthioxanthone, 4-isopropylthioxanthone, 2,3-diethylthioxanthone, 2,4-diethylthioxanthone, 2,4-dichlorothioxanthone, 2-methoxythioxanthone, 1-chloro-4-propoxythioxanthone, 2-cyclohexylthioxanthone, 4-cyclohexylthioxanthone, 2-vinylthioxanthone, 2,4-divinylthioxanthone, 2,4-diphenylthioxanthone, 2-butenyl-4-phenylthioxanthone, and 2-p-octyloxyphenyl-4-ethylthioxanthone. Preferred among these are those which are substituted at one or two, especially two, of R⁶ to R⁹ and R^{6'} to R^{9'} with alkyl groups. More preferred is 2,4-diethylthioxanthone.

The adsorption of a photopolymerization initiator such as a benzophenone or thioxanthone compound onto the outer surface and optionally the inner surface of the object may be carried out as follows. In the case of a benzophenone or thioxanthone compound, for example, the benzophenone or thioxanthone compound is dissolved in an organic solvent to prepare a solution; a surface portion of the object is treated with this solution so that the compound is adsorbed on the outer surface and optionally the inner surface; and, if necessary, the organic solvent is dried and evaporated off, whereby polymerization initiation points are formed on the outer surface and optionally the inner surface. The surface-treating method may be any method that allows the solution of the benzophenone or thioxanthone compound to be brought into contact with the outer surface and optionally the inner surface of the object. Suitable methods include applying or spraying the benzophenone or thioxanthone compound solution onto the surface; or immersing the surface into the solution. Moreover, the photopolymerization initiator may be adsorbed onto a part of the surface of the object. Examples of the solvent include methanol, ethanol, acetone, benzene, toluene, methyl ethyl ketone, ethyl acetate, and THF. Acetone is preferred because it does not swell the object intended to be modified, and it dries and evaporates quickly.

As described above, after the photopolymerization initiator is adsorbed on the outer surface and optionally the inner surface of the object, irradiation with ultraviolet light having a wavelength of 300 to 400 nm may further be performed to form polymerization initiation points from the photopolymerization initiator on the outer surface and optionally the inner surface. This ultraviolet light irradiation can be carried out by known methods. For example, it may be carried out as described for the ultraviolet light irradiation in step 2, which will be described later.

In step 2, the outer surface (the outer surface of the side wall) of the tubular object is irradiated with ultraviolet light having a wavelength of 300 to 400 nm from outside (outside of the side wall) to radically polymerize a monomer using radicals generated from the polymerization initiation points formed in step 1, to grow polymer chains on at least the inner surface (the inner surface of the side wall) of the tubular object. In particular, light irradiation of the outer surface generates radicals on the outer surface while simultaneously allowing part of the generated radicals to come around (transfer) to the inner surface of the tubular object, and further allows the transferred radicals to cause a radical polymerization from the inner surface so that polymer chains can be formed also on the inner surface. Due to this, polymer chains can be formed on the inner surface even when the object to be modified is opaque. Accordingly, polymer molecules can be sufficiently fixed not only to the outer surface but also to the inner surface of the tubular object, thereby imparting excellent lubricity and excellent lubricant durability to repeated movements.

Suitable examples of the monomer include alkali metal-containing monomers (monomers containing an alkali metal in the molecule), zwitterionic monomers (zwitterionic group-containing compounds: compounds bearing a center of permanent positive charge and a center of negative charge), and halogen-containing monomers (monomers containing a halogen in the molecule), which may be used alone or in combinations of two or more. If monomers simultaneously correspond to two or more of the above types, i.e. alkali metal-containing monomers, zwitterionic monomers, and halogen-containing monomers, as in the case of, for example, a monomer containing an alkali metal and a halogen (corresponding to both the alkali metal-containing monomer type and the halogen-containing monomer type), they are included in any of these two or more monomer types. The monomers may be used alone or in combinations of two or more.

Examples of the alkali metal-containing monomers include alkali metal salts of acrylic acid such as sodium acrylate and potassium acrylate; alkali metal salts of methacrylic acid such as sodium methacrylate and potassium methacrylate; alkali metal salts of itaconic acid such as sodium itaconate and potassium itaconate; alkali metal salts of 3-vinylpropionic acid such as sodium 3-vinylpropionate and potassium 3-vinylpropionate; alkali metal salts of vinylsulfonic acid such as sodium vinylsulfonate and potassium vinylsulfonate; alkali metal salts of 2-sulfoethyl (meth)acrylate such as sodium 2-sulfoethyl (meth)acrylate and potassium 2-sulfoethyl (meth)acrylate; alkali metal salts of 3-sulfopropyl (meth) acrylate such as sodium 3-sulfopropyl (meth) acrylate and potassium 3-sulfopropyl (meth) acrylate; alkali metal salts of 2-acrylamide-2-methylpropanesulfonic acid such as sodium 2-acrylamide-2-methylpropanesulfonate and potassium 2-acrylamide-2-methylpropanesulfonate; and alkali metal salts of styrenesulfonic acid such as sodium styrenesulfonate and potassium styrenesulfonate. Preferred among these is potassium 3-sulfopropyl methacrylate.

Examples of the zwitterionic monomers include carboxybetaines, sulfobetaines, and phosphobetaines. Compounds represented by the Formula (1) below may also be mentioned, and compounds represented by the Formula (2) below, among others, are suitable.

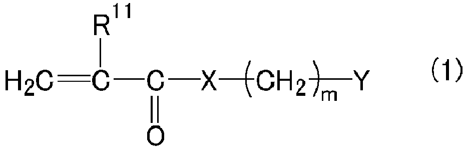

In the formula, R¹¹ represents -H or -CH₃; X represents -O-, -NH-, or -N⁺-; m represents an integer of 1 or larger; and Y represents a zwitterionic group or a halogen group such as Cl⁻, Br⁻, or F⁻.

In Formula (1), preferably, R¹¹ is -CH₃, X is -O-, and m is an integer of 1 to 10. In the zwitterionic group designated by Y, the cation may be a quaternary ammonium such as tetraalkylammonium, and the anion may be a carboxylate, sulfonate, or phosphate.

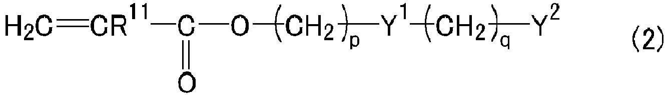

In the formula, R¹¹ represents -H or -CH₃; p and q each represent an integer of 1 or larger; and Y¹ and Y² represent ionic functional groups having electric charges opposite to each other.

In Formula (2), p is preferably an integer of 2 or larger, more preferably an integer of 2 to 10, and q is preferably an integer of 1 to 10, more preferably an integer of 2 to 4. Preferred examples of R¹¹ are the same as mentioned above. The symbols Y¹ and Y² are as described for the cation and anion above.

Typical suitable examples of the zwitterionic monomers include compounds represented by the following Formulas (2-1) to (2-4) : 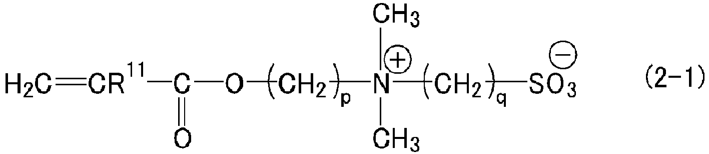 wherein R¹¹ represents a hydrogen atom or a methyl group, and p and q each represent an integer of 1 to 10, 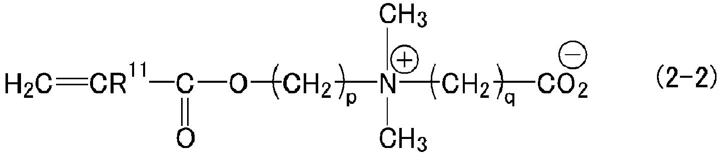 wherein R¹¹ represents a hydrogen atom or a methyl group, and p and q each represent an integer of 1 to 10, 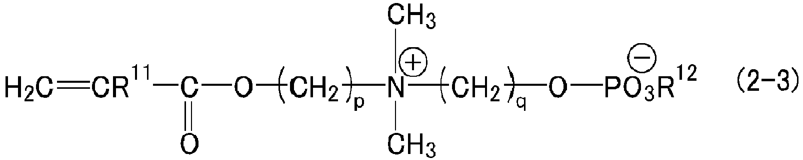 wherein R¹¹ represents a hydrogen atom or a methyl group; R¹² represents a C1-C6 hydrocarbon group; and p and q each represent an integer of 1 to 10, and 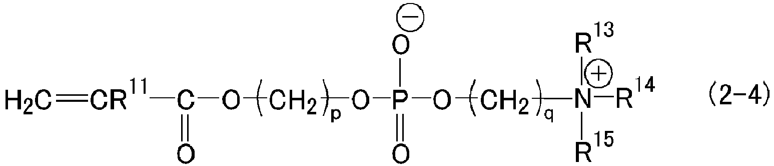 wherein R¹¹ represents a hydrogen atom or a methyl group; R¹³, R¹⁴, and R¹⁵ are the same as or different from one another and each represent a C1 or C2 hydrocarbon group; and p and q each represent an integer of 1 to 10.

Examples of compounds represented by Formula (2-1) include dimethyl(3-sulfopropyl)(2-(meth)acryloyloxyethyl) ammonium betaine. Examples of compounds represented by Formula (2-2) include dimethyl(2-carboxyethyl)-(2-(meth)acryloyloxyethyl)ammonium betaine. Examples of compounds represented by Formula (2-3) include dimethyl (3-methoxyphosphopropyl) (2-(meth)acryloyloxyethyl)ammonium betaine. Examples of compounds represented by Formula (2-4) include 2-(meth)acryloyloxyethyl phosphorylcholine. Other zwitterionic monomers include 2-(meth)acryloyloxyethyl carboxybetaine, and 2-(meth)acryloyloxyethyl sulfobetaine. Among these, 2-(meth)acryloyloxyethyl phosphorylcholine is particularly preferred because of its high biocompatibility, i.e. low protein adsorbability.

The term "halogen-containing monomer" refers to a monomer containing a halogen atom in the molecule. The halogen-containing monomers may be used alone or in combinations of two or more.

In view of lubricity and lubricant durability, the halogen-containing monomer may suitably be a nitrogen-containing monomer (halogen- and nitrogen-containing monomer). Specific preferred examples of such monomers include compounds represented by the following Formula (I): 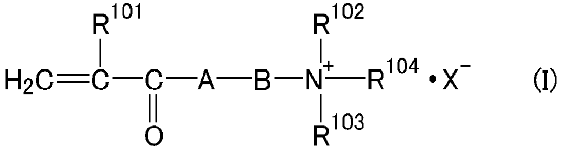 wherein A represents an oxygen atom or NH; B represents a C1-C4 alkylene group; R¹⁰¹ represents a hydrogen atom or a methyl group; R¹⁰², R¹⁰³, and R¹⁰⁴ are the same as or different from one another and each represent a C1-C4 alkyl group; and X represents a halogen ion.

The symbol A is preferably an oxygen atom. The symbol B may be a linear or branched alkylene group such as a methylene group, an ethylene group, or a propylene group, with a methylene group or an ethylene group being preferred among these. Each of R¹⁰² to R¹⁰⁴ may be a linear or branched alkyl group such as a methyl group, an ethyl group, or a propyl group, with a methyl group or an ethyl group being preferred among these. The symbol X (halogen atom) may be fluorine, chlorine, bromine or other halogens, preferably chlorine.

Examples of nitrogen-containing monomers represented by Formula (I) include 2-(methacroyloxy)ethyl trimethylammonium chloride (2-(methacroyloxy)ethyl trimethylaminium chloride), 2-(acryloyloxy)ethyl trimethylammonium chloride (2-(acryloyloxy)ethyl trimethylaminium chloride), 2-(methacroyloxy)ethyl dimethylethylammonium chloride (2-(methacroyloxy)ethyl dimethylethylaminium chloride), and 2-(acryloyloxy)ethyl dimethylethylammonium chloride (2-(acryloyloxy)ethyl dimethylethylaminium chloride).

The radical polymerization of a monomer in step 2 may be carried out, for example, as follows: a solution of a monomer or a liquid monomer is applied (sprayed) onto the outer surface and optionally the inner surface of the tubular object on which a benzophenone or thioxanthone compound or the like has been adsorbed, or the object is immersed in a solution of a monomer or a liquid monomer; and then the outer surface of the object is irradiated with ultraviolet light from outside to cause a radical polymerization (photoradical polymerization) on the outer surface using radicals generated on the outer surface, a radical polymerization (photoradical polymerization) on the inner surface using radicals transferred from the outer surface to the inner surface, and/or a radical polymerization (photoradical polymerization) on the inner surface using radicals generated on the inner surface, and the like, whereby polymer chains can be grown on the outer and inner surfaces of the object. After the application, the outer surface may further be covered with a transparent cover of glass, PET, polycarbonate or other materials, followed by irradiating the covered surface with ultraviolet light to allow the radical polymerizations (photoradical polymerizations) to proceed, whereby polymer chains can be grown on the outer and inner surfaces of the object.

The solvent for application (spraying), the method for application (spraying), the method for immersion, the conditions for irradiation, and other conditions may be conventionally known materials or methods. The solution of the radically polymerizable monomer may be an aqueous solution, or a solution in an organic solvent that does not dissolve the photopolymerization initiator (e.g. benzophenone or thioxanthone compound) used. Moreover, the solution of the radically polymerizable monomer or the liquid radically polymerizable monomer may contain a known polymerization inhibitor such as 4-methylphenol.

In the present invention, the radical polymerization of the monomer is allowed to proceed by light irradiation after the application of the solution of the monomer or the liquid monomer, or after the immersion in the solution of the monomer or the liquid monomer. In the light irradiation, ultraviolet light sources with an emission wavelength mainly in the ultraviolet region, such as high-pressure mercury lamps, metal halide lamps, and LED lamps, can be suitably used. The light dose may be selected appropriately in view of polymerization time and uniform progress of the reaction. Moreover, in order to prevent inhibition of polymerization due to active gas such as oxygen in the reaction vessel and the reaction pipe, oxygen is preferably removed from the reaction vessel, the reaction pipe, and the reaction solution during or before the light irradiation. To this end, appropriate operations may be performed; for example, an inert gas such as nitrogen gas or argon gas is introduced into the reaction vessel, the reaction pipe, and the reaction solution to discharge active gas such as oxygen from the reaction system and replace the atmosphere in the reaction system with the inert gas. Furthermore, in order to prevent inhibition of the reaction due to oxygen and the like, for example, a measure may appropriately be taken in which an ultraviolet light source is placed such that an air layer (oxygen content: 15% or higher) does not exist between the reaction vessel made of glass, plastics or the like and the reaction solution or the object to be modified.

The ultraviolet light has a wavelength of 300 to 400 nm. Such a wavelength enables polymer chains to be formed well on the outer and inner surfaces of the tubular object. Examples of light sources that can be used include high-pressure mercury lamps, LEDs with a center wavelength of 365 nm, LEDs with a center wavelength of 375 nm, and LEDs with a center wavelength of 385 nm. More preferred is irradiation with LED light having a wavelength of 355 to 390 nm. In particular, for example, LEDs with a center wavelength of 365 nm, which is close to the excitation wavelength (366 nm) of benzophenone, are preferred in view of efficiency. Light having a wavelength of less than 300 nm can cleave and damage molecules of the object intended to be modified. For this reason, light having a wavelength of 300 nm or greater is preferred. More preferred is light having a wavelength of 355 nm or greater because it produces very little damage to the object. In contrast, light having a wavelength of greater than 400 nm is less likely to activate the photopolymerization initiator, so that the polymerization reaction does not readily proceed. For this reason, light having a wavelength of 400 nm or less is preferred. Although LED light is suitable because the wavelength range of LED light is narrow so that no wavelengths other than the center wavelength are emitted, a mercury lamp or the like can also achieve similar effects to those of LED light if a filter is used to block light with wavelengths less than 300 nm.

In the present invention, the duration of irradiation with light having a wavelength of 300 to 400 nm can be shortened to achieve high productivity for forming polymer chains. For example, the duration of light irradiation can be reduced to 3 to 120 minutes, and even to 5 to 100 minutes or to 10 to 60 minutes.

The present invention also relates to a method for surface-modifying a tubular object made of a rubber vulcanizate or a thermoplastic elastomer, the method including step I of irradiating the outer surface of the object with ultraviolet light having a wavelength of 300 to 400 nm in the presence of a photopolymerization initiator to radically polymerize a monomer to grow polymer chains on at least the inner surface of the object. Specifically, the outer surface (the outer surface of the side wall) of the tubular object is irradiated with ultraviolet light in the presence of a photopolymerization initiator as a polymerization initiator to radically polymerize a monomer using radicals generated to grow polymer chains on at least the inner surface (the inner surface of the side wall) of the tubular object.

In particular, light irradiation of the outer surface generates radicals on the outer surface while simultaneously allowing part of the generated radicals to come around (transfer) to the inner surface of the tubular object, and further allows the transferred radicals to cause a radical polymerization from the inner surface so that polymer chains can be formed also on the inner surface. Due to this, polymer chains can be formed on the inner surface even when the object to be modified is opaque. Accordingly, polymer molecules can be sufficiently fixed not only to the outer surface but also to the inner surface of the tubular object, thereby imparting excellent lubricity and excellent lubricant durability to repeated movements. The tubular object to be modified, the photopolymerization initiator, and the monomer used in step I may be the same as those described above.

For example, in step I, a photopolymerization initiator and a monomer are brought into contact with the outer surface and optionally the inner surface of the tubular object to be modified, and then irradiation with LED light having a wavelength of 300 to 400 nm is performed to cause a radical polymerization (photoradical polymerization) on the outer surface using radicals generated on the outer surface, a radical polymerization (photoradical polymerization) on the inner surface using radicals transferred from the outer surface to the inner surface, and/or a radical polymerization (photoradical polymerization) on the inner surface using radicals generated on the inner surface, and the like, whereby polymer chains can be grown on the outer and inner surfaces of the object.

The radical polymerization of a monomer in step I may be carried out as follows: a solution of a monomer or a liquid monomer which contains a photopolymerization initiator such as a benzophenone or thioxanthone compound is applied (sprayed) onto the outer surface and optionally the inner surface of the tubular object to be modified, or the object is immersed in a solution of a monomer or a liquid monomer which contains a photopolymerization initiator; and then the outer surface of the object is irradiated with ultraviolet light from outside to allow the radical polymerizations (photoradical polymerizations) to proceed, whereby polymer chains can be grown on the outer and inner surfaces of the object. Further, for example, the outer surface may be covered with a transparent cover of glass, PET, polycarbonate or other materials, followed by irradiating the covered surface with ultraviolet light as described hereinabove. The solvent for application (spraying), the method for application (spraying), the method for immersion, the conditions for irradiation, and other conditions may be the materials or methods described hereinabove. Similarly to the above, the duration of irradiation with light having a wavelength of 300 to 400 nm may be reduced to 30 to 120 minutes, to 5 to 100 minutes, or to 10 to 60 minutes.

In step 2 or step I, two or more types of monomers may be radically polymerized simultaneously. Moreover, multiple types of polymer chains may be grown on the outer and inner surfaces of the tubular object to be modified. In the surface modification methods of the present invention, the polymer chains may be crosslinked to one another. In this case, the polymer chains may be crosslinked to one another by ionic crosslinking, crosslinking by a hydrophilic group containing an oxygen atom, or crosslinking by a halogen group such as iodine.

The above-described surface modification methods can be applied to rubber vulcanizate or thermoplastic elastomer tubes to produce surface-modified elastic bodies, for example, surface-modified elastic bodies that are excellent in lubricity in the presence of water. Preferred examples of the surface-modified elastic bodies include polymer brushes. The term "polymer brush" means an assembly of graft polymer molecules obtained in the "grafting from" approach by surface-initiated living radical polymerization. The graft chains are preferably oriented in a direction substantially vertical to the outer and inner surfaces of the tubular object because then the entropy decreases to reduce the molecular mobility of the graft chains, thereby providing lubricity. Furthermore, semidilute or concentrated brushes having a brush density of 0.01 chains/nm² or higher are preferred.

The surface modification methods may also be applied to rubber vulcanizate or thermoplastic elastomer tubes to produce medical devices, such as catheters, in which at least a part of the inner surface of the tubular object is modified. The modification is applied to the surface of medical devices such as catheters preferably at least at a portion that requires lubricity in the present of water, and may be applied to the entire surface.

### EXAMPLES

The present invention is more specifically described with reference to, but not limited to, examples below.

### (Example 1)

A 3 wt% solution of benzophenone in acetone was applied to the inner and outer surfaces of a black thermoplastic elastomer tube (material: nylon 6, length: 5 cm, inner diameter: 7 mm, outer diameter: 10 mm) so that benzophenone was adsorbed onto the surfaces, followed by drying.

Subsequently, the tube was immersed in an aqueous solution of potassium 3-sulfopropyl methacrylate (1.25 M) in a glass reaction vessel. The reaction vessel was sealed with a rubber stopper, and argon gas was introduced and allowed to bubble through the solution for 120 minutes to remove oxygen.

The glass reaction vessel was irradiated with LED light having a wavelength of 365 nm from outside of the tube for 45 minutes while being rotated to perform radical polymerization. Thus, polymer chains were grown on the inner and outer surfaces of the tube, whereby a surface-modified elastic body (polymer brush) was prepared.

### (Example 2)

A surface-modified elastic body (polymer brush) was prepared as in Example 1, except that benzophenone was replaced by 2,4-diethylthioxanthone and the duration of irradiation of LED light was changed from 45 minutes to 20 minutes.

### (Example 3)

A surface-modified elastic body (polymer brush) was prepared as in Example 1, except that potassium 3-sulfopropyl methacrylate was replaced by 2-methacryloyloxyethyl phosphorylcholine.

### (Example 4)

A surface-modified elastic body (polymer brush) was prepared as in Example 2, except that potassium 3-sulfopropyl methacrylate was replaced by 2-(methacroyloxy)ethyl trimethylammonium chloride.

### (Example 5)

A surface-modified elastic body (polymer brush) was prepared as in Example 3, except that the black thermoplastic elastomer tube (material: nylon 6, length: 5 cm, inner diameter: 7 mm, outer diameter: 10 mm) was replaced by a black thermoplastic elastomer tube (material: nylon 6, length: 10 cm, inner diameter: 7 mm, outer diameter: 10 mm).

### (Example 6)

A surface-modified elastic body (polymer brush) was prepared as in Example 3, except that the black thermoplastic elastomer tube (material: nylon 6, length: 5 cm, inner diameter: 7 mm, outer diameter: 10 mm) was replaced by a black thermoplastic elastomer tube (material: nylon 6, length: 30 cm, inner diameter: 7 mm, outer diameter: 10 mm).

### (Example 7)

A surface-modified elastic body (polymer brush) was prepared as in Example 3, except that the black thermoplastic elastomer tube (material: nylon 6, length: 5 cm, inner diameter: 7 mm, outer diameter: 10 mm) was replaced by a black thermoplastic elastomer tube (material: nylon 6, length: 5 cm, inner diameter: 1 mm, outer diameter: 2 mm).

### (Example 8)

A surface-modified elastic body (polymer brush) was prepared as in Example 1, except that the black thermoplastic elastomer tube (material: nylon 6, length: 5 cm, inner diameter: 7 mm, outer diameter: 10 mm) was replaced by a black thermoplastic elastomer tube (material: polyurethane, length: 5 cm, inner diameter: 7 mm, outer diameter: 10 mm).

### (Example 9)

A black thermoplastic elastomer tube (material: nylon 6, length: 5 cm, inner diameter: 7 mm, outer diameter: 10 mm) was immersed in an aqueous solution of potassium 3-sulfopropyl methacrylate (1.25 M) to which benzophenone was added and adjusted at a concentration of 0.003 M in a glass reaction vessel. The reaction vessel was sealed with a rubber stopper, and argon gas was introduced and allowed to bubble through the solution for 120 minutes to remove oxygen. The glass reaction vessel was irradiated with LED light having a wavelength of 365 nm for 90 minutes while being rotated to perform radical polymerization. Thus, polymer chains were grown on the inner and outer surfaces of the tube, whereby a surface-modified elastic body (polymer brush) was prepared.

### (Comparative Example 1)

A black thermoplastic elastomer tube (material: nylon 6, length: 5 cm, inner diameter: 7 mm, outer diameter: 10 mm) was used without modification.

### (Comparative Example 2)

The inner and outer surfaces of a black thermoplastic elastomer tube (material: nylon 6, length: 5 cm, inner diameter: 7 mm, outer diameter: 10 mm) was coated with a 5% solution of methyl vinyl ether-maleic anhydride (GANTREZ-AN 16, available from IPS) in methanol, and this coated tube was used. It should be noted that nylon is a material often used for vascular catheters, and methyl vinyl ether-maleic anhydride is a typical lubricant to impart lubricity to the surfaces.

The surface-modified elastic bodies prepared in the examples and the comparative examples were evaluated as follows and the results are shown in Table 1.

### (Lubricity)

The tube was cut to expose the inner and outer surfaces. Water was applied to the surfaces, and the sliding properties of the surfaces were subjectively evaluated by touching with a human finger. The subjective evaluation was carried out by ten persons according to a rating scale of 1-5, where a rating of 5 corresponds to a tube with good sliding properties, and a rating of 1 corresponds to a tube with poor sliding properties that do not allow the finger to slide on the surface. The average of the ratings was calculated.

### (Lubricant durability)

After water was applied to the inner and outer surfaces of the tube, the tube was held between fingers and slid on the fingers. This cycle was repeated 100 times. Then, the subjective evaluation was again carried out by ten persons according to the rating scale for lubricity, and the average of the ratings and the rate of decrease from the initial lubricity were calculated.

**[Table 1]**

| | | Example | | | | | | | | | Comparative Example | |
|---|---|---|---|---|---|---|---|---|---|---|---|---|
| | | 1 | 2 | 3 | 4 | 5 | 6 | 7 | 8 | 9 | 1 | 2 |
| Inner surface (end of tube) | Lubricity | 4.7 | 4.6 | 4.2 | 4.9 | 4.9 | 4.9 | 4.9 | 4.5 | 4.2 | 1 | 4.1 |
| | Durability | 4.6 | 4.5 | 3.9 | 4.8 | 4.8 | 4.8 | 4.8 | 4.4 | 4.1 | 1 | 2.2 |
| | Rate of decrease | 2.1% | 2.2% | 7.1% | 2.0% | 2.0% | 2.0% | 2.0% | 2.2% | 2.4% | 0% | 46.3% |
| Inner surface (center of tube) | Lubricity | 4.6 | 4.5 | 4.2 | 4.8 | 4.7 | 4.4 | 4.3 | 4.4 | 4.2 | 1 | 3.8 |
| | Durability | 4.5 | 4.4 | 3.9 | 4.7 | 4.6 | 4.3 | 4.2 | 4.3 | 4.1 | 1 | 2 |
| | Rate of decrease | 2.2% | 2.2% | 7.1% | 2.1% | 2.1% | 2.3% | 2.3% | 2.3% | 2.4% | 0% | 47.4% |
| Outer surface | Lubricity | 4.7 | 4.6 | 4.3 | 4.9 | 4.9 | 4.9 | 4.9 | 4.5 | 4.2 | 1 | 4.2 |
| | Durability | 4.6 | 4.5 | 4 | 4.8 | 4.8 | 4.8 | 4.8 | 4.4 | 4.1 | 1 | 2.4 |
| | Rate of decrease | 2.1% | 2.2% | 7.0% | 2.0% | 2.0% | 2.0% | 2.0% | 2.2% | 2.4% | 0% | 42.9% |

As shown in Table 1, the inner and outer surfaces of the tubes of the examples had high lubricity, good durability, and quite a low rate of decrease in lubricity. In contrast, the untreated tube of Comparative Example 1 exhibited very poor lubricity on both the inner and outer surfaces, and the commonly used product of Comparative Example 2 had moderately high initial lubricity but exhibited low durability and quite a high rate of decrease in lubricity.

The above results demonstrated that sufficient lubricity and sufficient lubricant durability can be simultaneously imparted not only to the outer surface but also to the inner surface of vascular catheters or other objects by forming polymer chains on the inner and outer surfaces from a monomer such as potassium 3-sulfopropyl methacrylate, 2-methacryloyloxyethyl phosphorylcholine, or 2-(methacroyloxy)ethyl trimethylammonium chloride.

### REFERENCE SIGNS LIST

- 11:: side wall
- 12:: opening
- 13:: interior

## Claims

1. A method of surface-modifying a tubular object made of a rubber vulcanizate or a thermoplastic elastomer whose side wall may have an opening, the method comprising:
step 1 of forming polymerization initiation points on at least an outer surface of the object; and
step 2 of irradiating the outer surface of the object with ultraviolet light having a wavelength of 300 to 400 nm to radically polymerizing a monomer using radicals generated from the polymerization initiation points to grow polymer chains on at least an inner surface of the object,
wherein the object is an opaque tubular object made of a rubber vulcanizate or a thermoplastic elastomer, and
in step 1, polymerization initiation points are formed on the inner surface as well as the outer surface of the object.

2. A method of surface-modifying a tubular object made of a rubber vulcanizate or a thermoplastic elastomer, the method comprising
step I of irradiating an outer surface of the object with ultraviolet light having a wavelength of 300 to 400 nm in the presence of a photopolymerization initiator to radically polymerize a monomer to grow polymer chains on at least an inner surface of the object,
wherein the object is an opaque tubular object made of a rubber vulcanizate or a thermoplastic elastomer, and
in step I, polymerization initiation points are formed on the inner surface as well as the outer surface of the object.

3. The method according to claim 1 or 2,
wherein in step 2 or step I, polymer chains are grown on the outer surface as well as the inner surface of the object.

4. The method according to claim 2,
wherein the photopolymerization initiator is at least one of a benzophenone compound or a thioxanthone compound.

5. The method according to any one of claims 1 to 4,
wherein the monomer is at least one selected from the group consisting of alkali metal-containing monomers, halogen-containing monomers, and zwitterionic monomers.

6. The method according to any one of claims 1 to 5,
wherein at least a part of the inner surface of the object is modified to impart lubricity in the presence of water.

7. The method according to any one of claims 1 to 6,
wherein the object is a catheter.

## Patentansprüche

1. Verfahren zur Oberflächenmodifikation eines aus einem Kautschukvulkanisat oder einem thermoplastischen Elastomer hergestellten rohrförmigen Gegenstands, dessen Seitenwand eine Öffnung aufweisen kann, wobei das Verfahren umfasst:
Schritt 1 eines Bildens von Polymerisationsinitiierungspunkten auf mindestens einer äußeren Oberfläche des Gegenstands; und
Schritt 2 eines Bestrahlens der äußeren Oberfläche des Gegenstands mit ultraviolettem Licht mit einer Wellenlänge von 300 bis 400 nm, um ein Monomer unter Verwendung von Radikalen, die von den Polymerisationsinitiierungspunkten erzeugt werden, radikalisch zu polymerisieren und somit Polymerketten auf mindestens einer inneren Oberfläche des Gegenstands wachsen zu lassen,
wobei der Gegenstand ein undurchsichtiger rohrförmiger Gegenstand ist, der aus einem Kautschukvulkanisat oder einem thermoplastischen Elastomer hergestellt ist, und
in Schritt 1 Polymerisationsinitiierungspunkte sowohl auf der inneren Oberfläche als auch auf der äußeren Oberfläche des Gegenstands gebildet werden.

2. Verfahren zur Oberflächenmodifikation eines aus einem Kautschukvulkanisat oder einem thermoplastischen Elastomer hergestellten rohrförmigen Gegenstands, wobei das Verfahren umfasst
Schritt I eines Bestrahlens einer äußeren Oberfläche des Gegenstands mit ultraviolettem Licht mit einer Wellenlänge von 300 bis 400 nm in Gegenwart eines Fotopolymerisationsinitiators, um ein Monomer radikalisch zu polymerisieren und somit Polymerketten auf mindestens einer inneren Oberfläche des Gegenstands wachsen zu lassen,
wobei der Gegenstand ein undurchsichtiger rohrförmiger Gegenstand ist, der aus einem Kautschukvulkanisat oder einem thermoplastischen Elastomer hergestellt ist, und
in Schritt I Polymerisationsinitiierungspunkte auf der inneren Oberfläche sowie der äußeren Oberfläche des Gegenstands gebildet werden.

3. Verfahren nach Anspruch 1 oder 2, wobei in Schritt 2 oder Schritt I Polymerketten sowohl auf der äußeren Oberfläche als auch auf der inneren Oberfläche des Gegenstands wachsen.

4. Verfahren nach Anspruch 2, wobei der Fotopolymerisationsinitiator mindestens eine von einer Benzophenonverbindung oder einer Thioxanthonverbindung ist.

5. Verfahren nach einem der Ansprüche 1 bis 4, wobei das Monomer mindestens eines ist, ausgewählt aus der Gruppe, bestehend aus Alkalimetall enthaltenden Monomeren, Halogen enthaltenden Monomeren und zwitterionischen Monomeren.

6. Verfahren nach einem der Ansprüche 1 bis 5, wobei mindestens ein Teil der inneren Oberfläche des Gegenstands modifiziert wird, um in Gegenwart von Wasser Schmierfähigkeit zu verleihen.

7. Verfahren nach einem der Ansprüche 1 bis 6, wobei der Gegenstand ein Katheter ist.

## Revendications

1. Procédé de modification de surface d'un objet tubulaire réalisé en vulcanisat de caoutchouc ou en un élastomère thermoplastique dont une paroi latérale peut avoir une ouverture, le procédé comprenant :
une étape 1 consistant à former des points de départ de polymérisation sur au moins une surface extérieure de l'objet ; et
une étape 2 consistant à irradier la surface extérieure de l'objet avec une lumière ultraviolette ayant une longueur d'onde de 300 à 400 nm pour polymériser par voie radicale un monomère utilisant des radicaux générés à partir des points de départ de polymérisation pour faire croître des chaînes de polymère sur au moins une surface intérieure de l'objet,
dans lequel l'objet est un objet tubulaire opaque réalisé en vulcanisat de caoutchouc ou en un élastomère thermoplastique, et
dans l'étape 1, les points de départ de polymérisation sont formés sur la surface intérieure ainsi que sur la surface extérieure de l'objet.

2. Procédé de modification de surface d'un objet tubulaire réalisé en vulcanisat de caoutchouc ou en un élastomère thermoplastique, le procédé comprenant :
une étape 1 consistant à irradier une surface extérieure de l'objet avec une lumière ultraviolette ayant une longueur d'onde de 300 à 400 nm en présence d'un initiateur de photopolymérisation pour polymériser par voie radicale un monomère pour faire croître des chaînes de polymère sur au moins une surface intérieure de l'objet,
dans lequel l'objet est un objet tubulaire opaque réalisé en vulcanisat de caoutchouc ou en un élastomère thermoplastique, et
dans l'étape 1, des points de départ de polymérisation sont formés sur la surface intérieure ainsi que sur la surface extérieure de l'objet.

3. Procédé selon la revendication 1 ou 2,
dans lequel, dans l'étape 2 ou dans l'étape 1, des chaînes polymères sont amenées à croître sur la surface extérieure ainsi que sur la surface intérieure de l'objet.

4. Procédé selon la revendication 2,
dans lequel l'initiateur de photopolymérisation est au moins un composé parmi un composé benzophénone et un composé thioxanthone.

5. Procédé selon l'une quelconque des revendications 1 à 4,
dans lequel le monomère est au moins un monomère sélectionné parmi le groupe comprenant les monomères contenant un métal alcalin, les monomères contenant des halogènes, et des monomères zwitterioniques.

6. Procédé selon l'une quelconque des revendications 1 à 5,
dans lequel au moins une partie de la surface intérieure de l'objet est modifiée pour conférer une lubricité en présence d'eau.

7. Procédé selon l'une quelconque des revendications 1 à 6,
dans lequel l'objet est un cathéter.
